# EUROPÄISCHE PATENTSCHRIFT

(11) **EP 3 603 744 B1**
(45) Veröffentlichungstag und Bekanntmachung des Hinweises auf die Patenterteilung: **25.02.2026**
(21) Anmeldenummer: 18187094.0
(22) Anmeldetag: 02.08.2018
(51) Int. Cl.: A61N 1/375, A61N 1/362, H01R 13/504, H01R 43/18

(54) **BAUGRUPPE FÜR EINEN HEADER, HEADER UND IMPLANTAT MIT DEM HEADER SOWIE VERFAHREN ZUM MONTIEREN EINES HEADERS**
SUBASSEMBLY FOR A HEADER, HEADER AND IMPLANT COMPRISING THE HEADER AND METHOD FOR MOUNTING A HEADER
MODULE POUR UNE TÊTE, TÊTE ET IMPLANT POURVU DE LA TÊTE AINSI QUE PROCÉDÉ DE MONTAGE D'UNE TÊTE

(43) Veröffentlichungstag der Anmeldung: 05.02.2020
(73) Patentinhaber: BIOTRONIK SE & Co. KG, 12359 Berlin (DE)
(72) Erfinder: Ruschel, Marina, 12679 Berlin (DE); Hartmann-Bax, Kathy, 14947 Nuthe-Urstromtal (DE)
(74) Vertreter: Biotronik Corporate Services SE

(56) Entgegenhaltungen:
- EP-A2- 2 082 780
- US-A1- 2007 087 637
- US-A1- 2012 215 288
- US-A1- 2015 094 792
- US-A1- 2017 266 451
- US-A1- 2017 279 238

## Beschreibung

Die Offenbarung betrifft eine Baugruppe für eine Elektrodenanschlussvorrichtung (auch Header genannt), eine Elektrodenanschlussvorrichtung, ein Implantat mit der Elektrodenanschlussvorrichtung sowie ein Verfahren zum Montieren einer Elektrodenanschlussvorrichtung auf einem Implantat.

### Hintergrund

Bisher werden Header für aktive Implantate aus vielen einzelnen Komponenten aufgebaut. Wichtig ist hierbei, dass die Komponenten exakt zueinander ausgerichtet sind, bevor sie mit einem Harz vergossen werden, um den Header zu bilden. Dieses Verfahren ist sehr aufwändig.

Das Dokument US 2017/0266,451 A1 offenbart einen Header mit einer modularen Durchführung.

Das Dokument US 2017/0279,238 A1 offenbart einen Header mit einem vorgefertigten Header-Modul.

Ein weiterer Header ist in dem Dokument WO 2009/009299 A1 beschrieben.

Das Dokument US 2007/0087637 A1 offenbart einen Header mit einer Kontaktbuchsen-Baugruppe.

### Zusammenfassung

Aufgabe ist es verbesserte Technologien für Implantate anzugeben. Insbesondere soll die Herstellung der Elektrodenanschlussvorrichtung vereinfacht werden.

Es sind eine Baugruppe für eine Elektrodenanschlussvorrichtung eines Implantats nach Anspruch 1, eine Elektrodenanschlussvorrichtung nach Anspruch 7, ein Implantat nach Anspruch 8 und ein Verfahren zum Bilden einer Elektrodenanschlussvorrichtung auf einem Implantat nach Anspruch 9 offenbart. Weitere Ausführungsformen sind Gegenstand von abhängigen Ansprüchen.

Es ist eine Baugruppe für eine Elektrodenanschlussvorrichtung eines Implantats bereitgestellt. Die Baugruppe umfasst eine durchgehende Aufnahmeeinrichtung für einen Stecker. Des Weiteren ist ein erstes Anschlusselement vorgesehen, das in einem vorderen Bereich der Aufnahmeeinrichtung angeordnet ist, wobei das erste Anschlusselement wenigstens zwei ebene Seitenflächen aufweist. Schließlich ist ein zweites Anschlusselement vorgesehen, das in einem hinteren Bereich der Aufnahmeeinrichtung angeordnet ist, wobei das zweite Anschlusselement wenigstens zwei ebene Seitenflächen aufweist. Weiterhin ist die Baugruppe zumindest abschnittsweise von einem Kunststoff umgeben, wobei ein Anschlussbereich des ersten Anschlusselements frei von Kunststoff ist, und ein Anschlussbereich des zweiten Anschlusselements frei von Kunststoff ist. Weiterhin ist an einem hinteren Ende der Aufnahmeeinrichtung eine Positioniereinrichtung gebildet.

Des Weiteren ist eine Elektrodenanschlussvorrichtung für ein Implantat mit einer hier offenbarten Baugruppe bereitgestellt.

Die Offenbarung umfasst des Weiteren ein Implantat mit einer Elektrodenanschlussvorrichtung und einer Baugruppe.

Die Elektrodenanschlussvorrichtung kann auch Anschlusskopf oder Header genannt werden.

Die ebenen Seitenflächen ermöglichen eine zumindest teilweise eckige Form und erlauben ein einfaches Greifen der Baugruppe (manuell oder automatisch). Hierdurch kann eine Automatisierung des Herstellungsprozesses ermöglicht werden.

Wie oben beschrieben, ist die Baugruppe zumindest abschnittsweise von einem Kunststoff umgeben. Beispielsweise kann die Baugruppe von dem Kunststoff abschnittsweise umspritzt sein. Der Kunststoff kann ein Thermoplast sein, beispielsweise Polysulfon. Es kann auch ein biokompatibles Gießharz verwendet werden. Der Kunststoff kann der Baugruppe zusätzliche Stabilität verleihen. Hiermit ist es möglich, die Baugruppe als vorgefertigte Komponente herzustellen, welche anschließend zu einer Elektrodenanschlussvorrichtung eines Implantats verarbeitet wird.

In dem Kunststoff kann eine erste Führung für einen ersten Leiter zum Anschluss an den Anschlussbereich des ersten Anschlusselements gebildet sein und / oder in dem Kunststoff kann eine zweite Führung für einen zweiten Leiter zum Anschluss an den Anschlussbereich des zweiten Anschlusselements gebildet sein.

Die erste Führung kann benachbart zu dem Anschlussbereich des ersten Anschlusselements gebildet sein und / oder die zweite Führung kann benachbart zu dem Anschlussbereich des zweiten Anschlusselements gebildet sein.

An den Anschlussbereich des ersten Anschlusselements (des zweiten Anschlusselements) kann an erster Leiter (zweiter Leiter) angeschlossen werden, um eine Verbindung von einem in die Aufnahmeeinrichtung eingeführten Stecker zu einem Implantat zu ermöglichen. Der Anschlussbereich des ersten Anschlusselements und / oder der Anschlussbereich des zweiten Anschlusselements können als flächige Elemente ausgeführt sein. Der Anschlussbereich des ersten Anschlusselements und / oder der Anschlussbereich des zweiten Anschlusselements können kreisförmig gebildet sein und beispielsweise einen Durchmesser von 1 bis 5 mm haben. Hierdurch ist eine große Schweißfläche zum Befestigen des ersten Leiters bzw. des zweiten Leiters gegeben. In einer Ausführungsform sind sowohl die erste Führung als auch die zweite Führung benachbart zu den jeweiligen Anschlussbereichen gebildet. Die Führungen ermöglichen einen Anschluss der Leiter an die jeweiligen Anschlussbereiche, ohne einen Kurzschluss zu verursachen.

Es kann vorgesehen sein, dass das erste Anschlusselement und das zweite Anschlusselement zueinander versetzt angeordnet sind. Mit anderen Worten, das erste Anschlusselement und das zweite Anschlusselement liegen auf zwei verschiedenen Ebenen. Die unterschiedliche Anordnung erleichtert es, die Leiter an die Anschlusselemente anzuschließen, ohne dass die Leiter miteinander in Kontakt kommen.

In einer Ausführungsform kann die Baugruppe eine Antenne aufweisen, wobei die Antenne in einem Zwischenbereich, der zwischen dem ersten Anschlusselement und dem zweiten Anschlusselement gebildet ist, einen U-förmigen Verlauf hat. Der Zwischenbereich kann schmaler ausgebildet sein als die angrenzenden Anschlusselemente. Zusammen mit dem U-förmigen Verlauf der Antenne ist hierdurch eine Greifmulde für einen automatischen Greifer gebildet.

Die Positioniereinrichtung, die an einem hinteren Ende der Aufnahmeeinrichtung gebildet ist, kann als abgewinkelte Struktur gebildet sein und beispielsweise einen rechten Winkel zu der Aufnahmeeinrichtung bilden. Die Positioniereinrichtung kann aus dem Kunststoff gebildet sein und beispielsweise mit dem Kunststoffüberzug der Baugruppe einteilig gebildet sein. Die Positioniereinrichtung kann beim Anordnen der Baugruppe in eine Aufnahme an einem Gehäuse des Implantats angeordnet werden, um das Ausrichten der Baugruppe zu erleichtern. Die Positioniereinrichtung kann ein spitz zulaufendes Ende haben.

Die Baugruppe kann eine weitere Aufnahmeeinrichtung für einen weiteren Stecker aufweisen, wobei in einem vorderen Bereich der weiteren Aufnahmeeinrichtung ein drittes Anschlusselement angeordnet ist, und wobei in einem hinteren Bereich der weiteren Aufnahmeeinrichtung ein viertes Anschlusselement angeordnet ist. Für die weitere Aufnahmeeinrichtung gelten die hier offenbarten Ausführungen zu der Aufnahmeeinrichtung analog. Des Weiteren gelten für das dritte Anschlusselement und das vierte Anschlusselement die Ausführungen zum ersten Anschlusselement und zum zweiten Anschlusselement analog.

Nach einem weiteren Aspekt ist ein Verfahren zum Bilden einer Elektrodenanschlussvorrichtung auf einem Implantat offenbart. Das Verfahren umfasst folgende Schritte:
- Bereitstellen der oben offenbarten Baugruppe,
- Anordnen und Befestigen eines Federelements in der Aufnahmeeinrichtung,
- Verschließen von Öffnungen der Aufnahmeeinrichtung mit Vergusshilfsmitteln,
- Befestigen eines ersten Leiters an dem ersten Anschlusselement,
- Befestigen eines zweiten Leiters an dem zweiten Anschlusselement,
- Anordnen der Baugruppe auf einem Gehäuse des Implantats,
- Verbinden des ersten Leiters mit einer Durchführung, die an einem Gehäuse gebildet ist,
- Verbinden des zweiten Leiters mit der Durchführung,
- Anordnen der Baugruppe mit dem Gehäuse in einer Gießform,
- Füllen der Gießform mit einem Kunstharz,
- nach Aushärten des Kunstharzes, Entfernen der Vergusshilfsmittel.

Das Verfahren kann die weiteren Schritte umfassen:
- Anordnen und Befestigen einer Antenne an der Baugruppe,
- Verbinden der Antenne mit der Durchführung,
wobei die weiteren Schritte ausgeführt werden, bevor die Baugruppe auf dem Gehäuse angeordnet wird.

Des Weiteren kann vorgesehen sein, nach dem Aushärten eventuell überstehendes Harz zu entfernen, beispielsweise mittels Schleifen und / oder Polieren.

Die Gießform kann eine Silikonform sein.

Die Durchführung kann einen oder mehrere Steckkontakte (z.B. Stifte) zum Anschluss der Leiter und / oder der Antenne aufweisen.

Das Kunstharz kann ein Epoxidharz sein. Epoxidharze sind Kunstharze, die Epoxidgruppen tragen. Sie sind härtbare Harze (Reaktionsharze), die mit einem Härter und gegebenenfalls weiteren Zusatzstoffen zu einem duroplastischen Kunststoff umgesetzt werden können. Epoxidharze sind Polyether mit zwei endständigen Epoxidgruppen. Die Härtungsmittel sind Reaktionspartner und bilden zusammen mit dem Harz einen makromolekularen Kunststoff.

Das Kunstharz kann direkt auf dem Gehäuse des Implantats haften, so dass ein zusätzliches Haftmittel nicht erforderlich ist. Mit anderen Worten, die Kontaktfläche zwischen dem ausgehärteten Kunstharz und dem Gehäuse des Implantats kann frei von einem Haftmittel sein. Das Gehäuse des Implantats kann aus Titan bestehen.

Die Elektrodenanschlussvorrichtung kann ein Header für einen implantierbaren Herzschrittmacher oder einen implantierbaren Kardioverter-Defibrillator (ICD - Implantable Cardioverter Defibrillator) sein. In diesem Fall dient die Elektrodenanschlussvorrichtung zur elektrischen Verbindung von einer oder mehreren Elektrodenleitungen mit dem Implantat.

Die Merkmale, welche in Verbindung mit der Baugruppe und der Elektrodenanschlussvorrichtung offenbart sind, können in analoger Weise auf das Verfahren angewendet werden und anders herum.

### Beschreibung von Ausführungsbeispielen

Im Folgenden werden beispielhafte Ausführungsformen unter Bezugnahme auf Figuren näher erläutert. Es zeigen:
- Fig. 1: eine perspektivische Ansicht einer Ausführungsform einer Baugruppe für eine Elektrodenanschlussvorrichtung,
- Fig. 2: eine Vorderansicht (oberer Abschnitt von Fig. 2) und eine Hinteransicht (unterer Abschnitt von Fig. 2) der Baugruppe nach Fig. 1,
- Fig. 3: die Baugruppe nach Fig. 1 und 2 mit Vergusshilfsmitteln,
- Fig. 4: die Baugruppe nach Fig. 1 bis 3 mit einer Antenne und Leitern,
- Fig. 5: die Baugruppe nach Fig. 1 bis 4 auf einem Gehäuse angeordnet,
- Fig. 6: die Baugruppe nach Fig. 1 bis 5 vollständig vergossen (mit Vergusshilfsmitteln) und
- Fig. 7: die Baugruppe nach Fig. 1 bis 5 vollständig vergossen (ohne Vergusshilfsmittel).

Im Folgenden werden für gleiche Komponenten gleiche Bezugszeichen verwendet.

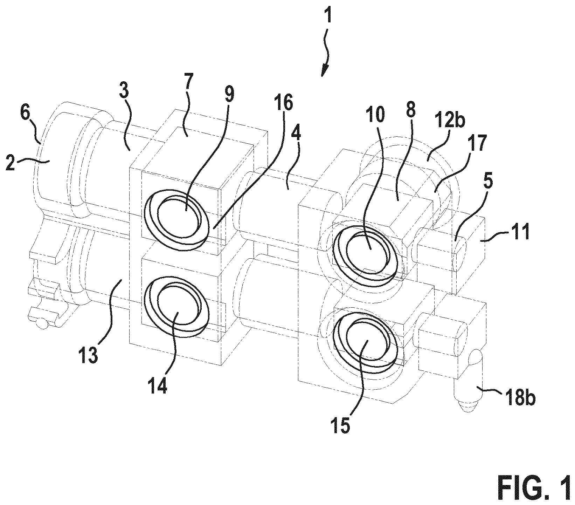

In den Fig. 1 bis 7 sind die einzelnen Schritte zur Montage einer Elektrodenanschlussvorrichtung (Header) auf einem Implantat dargestellt. Die Schritte werden im Folgenden näher erläutert.

Fig. 1 zeigt eine Baugruppe 1 (auch als Headerkern bezeichnet) mit einer ersten Aufnahmeeinrichtung 2 für einen Elektrodenstecker und einer zweiten Aufnahmeeinrichtung 13 für einen weiteren Elektrodenstecker. Die erste Aufnahmeeinrichtung 2 weist eine vordere Öffnung 6 auf, durch welche der Elektrodenstecker eingeführt werden kann. Die erste Aufnahmeeinrichtung 2 weist einen ersten Abschnitt 3, einen zweiten Abschnitt 4 und einen dritten Abschnitt 5 auf. Der Durchmesser des ersten Abschnitts 3 ist größer als der Durchmesser des zweiten Abschnitts 4. Der Durchmesser des zweiten Abschnitts 4 ist wiederum größer als der Durchmesser des dritten Abschnitts 5. Mit anderen Worten: die erste Aufnahmeeinrichtung 2 ist von der vorderen Öffnung 6 zum Ende hin stufenweise verjüngt.

Zwischen dem ersten Abschnitt 3 und dem zweiten Abschnitt 4 (also in einem vorderen Bereich der ersten Aufnahmeeinrichtung 2) ist ein erstes Anschlusselement gebildet. Zwischen dem zweiten Abschnitt 4 und dem dritten Abschnitt 5 (in einem hinteren Bereich der ersten Aufnahmeeinrichtung 2) ist ein zweites Anschlusselement 8 gebildet. Sowohl das erste Anschlusselement 7 als auch das zweite Anschlusselement 8 weisen mindestens zwei ebene Seitenflächen auf. Dies ermöglicht ein einfaches Greifen der Baugruppe 1 während einer Montage und ermöglicht eine Automatisierung der Montageschritte. In der dargestellten Ausführungsform sind das erste Anschlusselement 7 und das zweite Anschlusselement 8 im Wesentlichen quaderförmig gebildet. Das zweite Anschlusselement 8 weist eine abgeschrägte Kante 17 auf, welche zur Materialeinsparung und zur Beachtung der Fließrichtung des Epoxidharzes dient. An einer Rückseite des ersten Anschlusselements 7 ist eine Aussparung 12a in dem Kunststoffüberzug gebildet. An einer Rückseite des zweiten Anschlusselements 8 ist eine hintere Öffnung 12b gebildet.

Die Baugruppe ist zum Teil von einem Kunststoff 11 umgeben. In der dargestellten Ausführungsform ist die Baugruppe teilweise mit Polysulfon umspritzt. In dem Kunststoff 11 sind Aussparungen für eine erste Kontaktfläche 9 und eine zweite Kontaktfläche 10 gebildet. Die erste und zweite Kontaktfläche sind als kreisförmige Flächen gebildet. Benachbart zu der ersten und zweiten Kontaktfläche 9, 10 ist jeweils eine Führung 16 gebildet. Die Führung 16 dient zur Aufnahme eines Anschlusselements (z.B. eines Verdrahtungsbandes). Die Führungen an den Kontaktflächen verhindern, dass Anschlusselemente von verschiedenen Kontaktflächen einander berühren.

Die zweite Aufnahmeeinrichtung 13 ist analog zu der ersten Aufnahmeeinrichtung 2 aufgebaut. Aus Gründen der Übersichtlichkeit sind die Komponenten der zweiten Aufnahmeeinrichtung (eine Öffnung, die drei stufenförmig verjüngenden Abschnitte sowie die zwei Anschlusselemente) nicht mit Bezugszeichen versehen. Die zweite Aufnahmeeinrichtung weist ebenfalls zwei Kontaktflächen (dritte Kontaktfläche 14 und vierte Kontaktfläche 15) für Anschlüsse auf. Benachbart zu den Kontaktflächen sind wiederum Führungen gebildet.

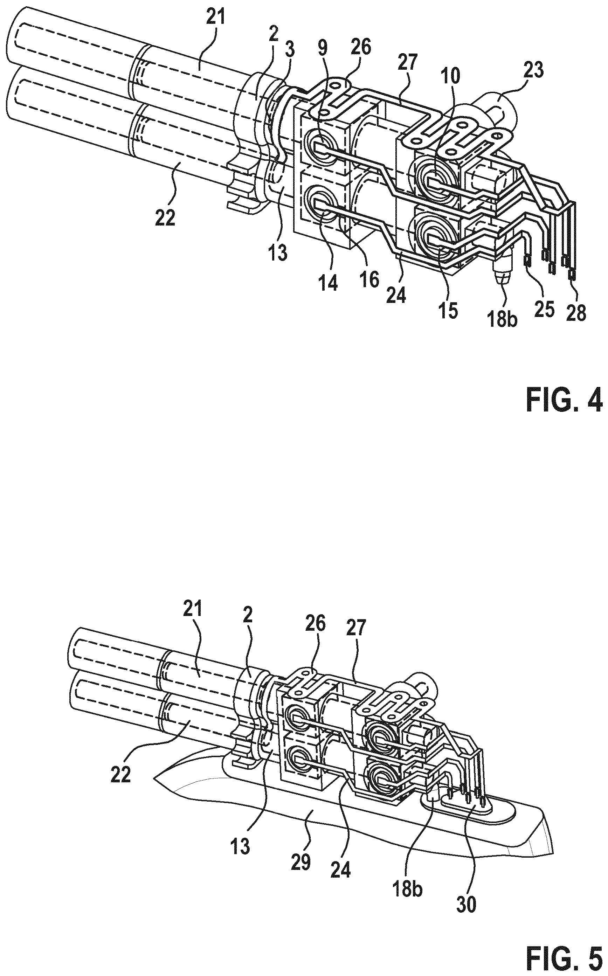

An einem Ende der zweiten Aufnahmeeinrichtung ist eine Positioniereinrichtung 18b gebildet, welche als Zapfen mit einem spitz zulaufenden Ende ausgeführt ist. Beim Montieren der Baugruppe 1 auf einem Gehäuse 29 (vgl. Fig. 5) kann das spitze Ende der Positioniereinrichtung 18b in eine Aufnahme des Gehäuses eingesetzt werden, um das passgenaue Anordnen der Baugruppe auf dem Gehäuse zu erleichtern. Die Baugruppe kann jedoch auch ohne Positioniereinrichtung 18b ausgeführt sein.

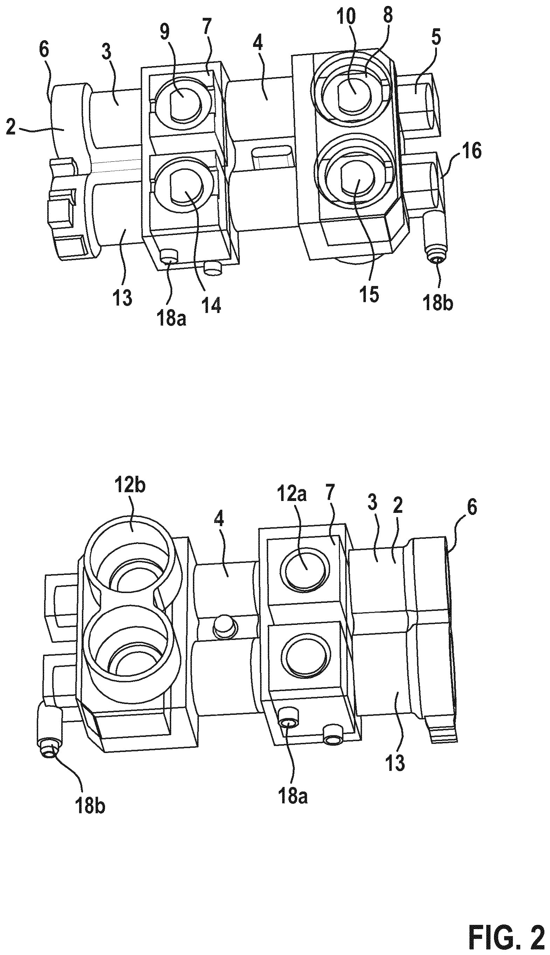

An einer Unterseite der Baugruppe sind Positionierstifte 18a gebildet (vgl. Fig. 2). Die Positionierstifte können beim Anordnen der Baugruppe auf dem Gehäuse des Implantats in zugeordneten Aufnahmen angeordnet werden. In der gezeigten Ausführungsform sind zwei Positionierstifte gezeigt, andere Zahlen von Positionierstiften sind jedoch möglich.

Die erste Aufnahmeeinrichtung 2 und die zweite Aufnahmeeinrichtung 13 weisen jeweils eine Federhülse und eine Steckeraufnahme auf. Die erste Aufnahmeeinrichtung 2 und die zweite Aufnahmeeinrichtung 13 können als IS-1 Konnektoren ausgebildet sein.

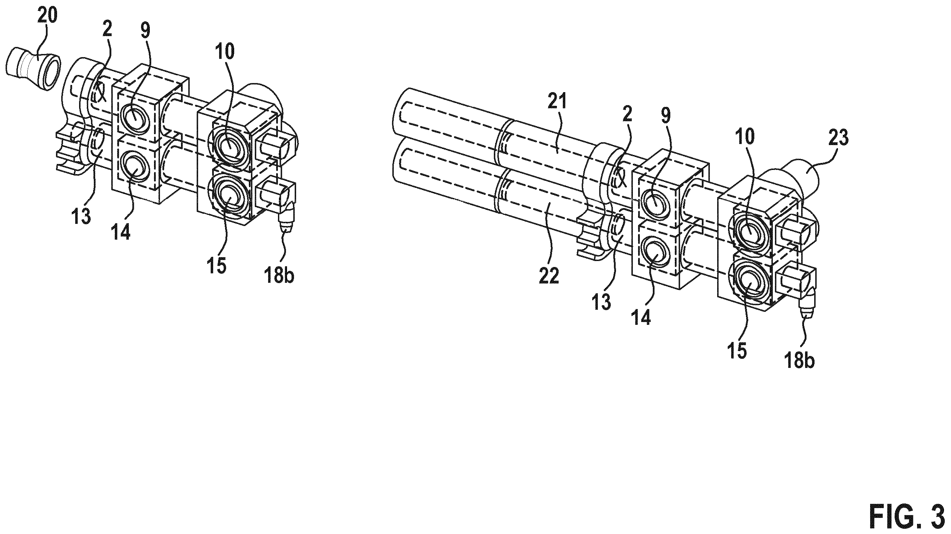

Ein Federelement 20 wird in der ersten Aufnahmeeinrichtung 2 angeordnet und dort befestigt (linke Seite von Fig. 3). Die Aussparung 12a in dem Kunststoff wird genutzt, um das im Inneren der ersten Aufnahmeeinrichtung 2 angeordnete Federelement 20 mittels Widerstandsschweißen in das erste Anschlusselement 7 einzuschweißen. Analog wird ein weiteres Federelement in der zweiten Aufnahmeeinrichtung 13 angeordnet und befestigt (nicht dargestellt). Die Öffnungen der Baugruppe 1 werden anschließend mit Vergusshilfsmitteln 21, 22, 23 verschlossen und abgedichtet (rechte Seite von Fig. 3).

In Fig. 4 ist ein weiterer Montageschritt gezeigt. Ein Drahtband 24 ist an der dritten Kontaktfläche 14 befestigt (z.B. verschweißt). An seinem hinteren Ende weist das Drahtband 24 einen Drahtbandanschluss 25 auf, der mit einem Stiftkontakt einer Durchführung 30 (vgl. Fig. 4) verbindbar ist und beispielsweise auf den Stiftkontakt aufgesteckt werden kann. Weitere Drahtbänder sind mit den anderen Kontaktflächen 9, 10, 15 verbunden.

An der Baugruppe 1 ist eine Antenne 26 befestigt. Die Antenne 26 umgreift den ersten Abschnitt 3 der ersten Aufnahmeeinrichtung 2 teilweise und ist hieran geklippt. In einem Bereich zwischen dem ersten Anschlusselement 7 und dem zweiten Anschlusselement 8 weist die Antenne 26 einen U-förmigen Abschnitt 27 auf. Hierdurch ist eine Griffmulde gebildet, die beispielsweise mit einem automatisierten Greifer verwendet werden kann, um die Baugruppe zu halten und zu transportieren. An einem hinteren Ende der Antenne ist ein Antennenanschluss 28 zur Verbindung mit der Durchführung 30 gebildet.

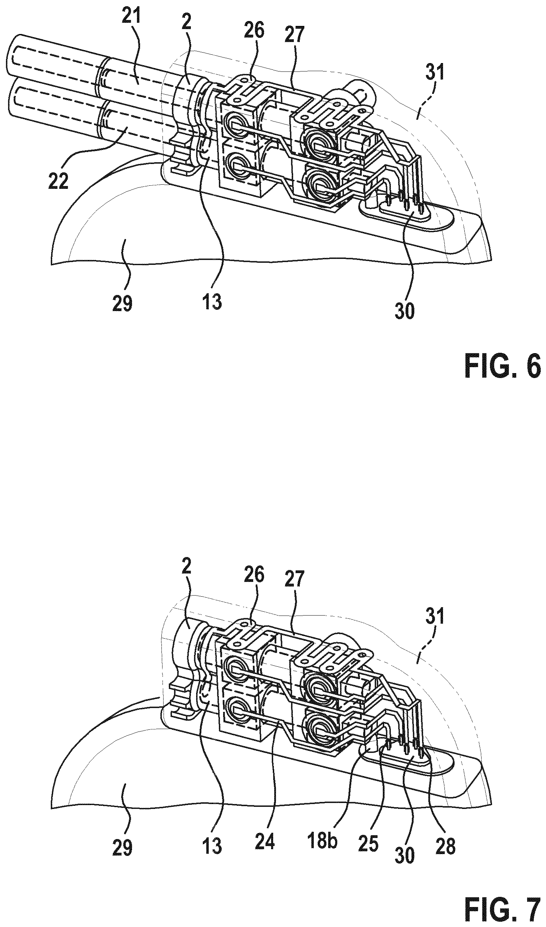

Die Baugruppe mit den Drahtbändern und der Antenne wird anschließend in einer Gießform (z.B. eine Silikonform) angeordnet (nicht dargestellt). Die Drahtbandanschlüsse und der Antennenanschluss 28 werden auf zugeordnete Stifte der Durchführung 30 gesteckt und mit den Stiften verbunden (z.B. verschweißt). Die Gießform wird verschlossen und mit einem Kunstharz 31 (z.B. Epoxidharz) gefüllt. Hierdurch wird die Elektrodenanschlussvorrichtung abgeformt (vgl. Fig. 6).

Die Vergusshilfsmittel 21, 22, 23 werden entfernt und ggf. überschüssiges Harz an den Außenflächen entfernt, z.B. mittels Schleifen und / oder Polieren. Das Implantat mit der Elektrodenanschlussvorrichtung ist hiermit fertig montiert (Fig. 7).

Die Baugruppe / Elektrodenanschlussvorrichtung kann folgenden Vorteile aufweisen:
- kein Eindringen von Harz und / oder anderen Partikeln in die erste (und ggf. zweite) Aufnahmeeinrichtung, daher ist eine Nacharbeit nicht erforderlich;
- Isolationsabstände der Drahtbänder sind durch das Design der Baugruppe vorgegeben, sodass das Risiko eines elektrischen Kurzschlusses verringert oder sogar ausgeschlossen ist;
- das Design ermöglicht eine automatisierte Herstellung der Elektrodenanschlussvorrichtung;
- gegenüber der bisher bekannten Fertigung entfallen Fertigungsschritte, was zu einer Verkürzung der Fertigungszeit führt.

Die in der Beschreibung, den Ansprüchen und den Figuren offenbarten Merkmale können für die Verwirklichung von Ausführungsformen sowohl einzeln als auch in beliebiger Kombination miteinander relevant sein.

### Bezugszeichenliste:

- 1: Baugruppe
- 2: erste Aufnahmeeinrichtung
- 3: erster Abschnitt der ersten Aufnahmeeinrichtung
- 4: zweiter Abschnitt der ersten Aufnahmeeinrichtung
- 5: dritter Abschnitt der ersten Aufnahmeeinrichtung
- 6: vordere Öffnung
- 7: erstes Anschlusselement
- 8: zweites Anschlusselement
- 9: erste Kontaktfläche
- 10: zweite Kontaktfläche
- 11: Kunststoff
- 12a: Aussparung
- 12b: hintere Öffnung
- 13: zweite Aufnahmeeinrichtung
- 14: dritte Kontaktfläche
- 15: vierte Kontaktfläche
- 16: Führung
- 17: abgeschrägte Kante
- 18a: Positionierstifte
- 18b: Positioniereinrichtung
- 20: Federelement
- 21: erstes Vergusshilfsmittel
- 22: zweites Vergusshilfsmittel
- 23: drittes Vergusshilfsmittel
- 24: Drahtband
- 25: Drahtbandanschluss
- 26: Antenne
- 27: U-förmiger Abschnitt der Antenne
- 28: Antennenanschluss
- 29: Gehäuse
- 30: Durchführung
- 31: Harz

## Patentansprüche

1. Baugruppe (1) für eine Elektrodenanschlussvorrichtung eines Implantats, mit
- einer durchgehenden Aufnahmeeinrichtung (2) für einen Stecker,
- einem ersten Anschlusselement (7), das in einem vorderen Bereich der Aufnahmeeinrichtung (2) angeordnet ist, wobei das erste Anschlusselement (7) wenigstens zwei ebene Seitenflächen aufweist, und
- einem zweiten Anschlusselement (8), das in einem hinteren Bereich der Aufnahmeeinrichtung (2) angeordnet ist, wobei das zweite Anschlusselement (8) wenigstens zwei ebene Seitenflächen aufweist.
wobei
die Baugruppe (1) zumindest abschnittsweise von einem Kunststoff (11) umgeben ist, und ein Anschlussbereich (9) des ersten Anschlusselements (7) frei von Kunststoff (11) ist und ein Anschlussbereich (10) des zweiten Anschlusselements (8) frei von Kunststoff (11) ist, und
an einem hinteren Ende der Aufnahmeeinrichtung eine Positioniereinrichtung (18b) gebildet ist.

2. Baugruppe (1) nach Anspruch 1, wobei in dem Kunststoff (11) eine erste Führung für einen ersten Leiter zum Anschluss an den Anschlussbereich (9) des ersten Anschlusselements (7) gebildet ist und / oder wobei in dem Kunststoff (11) eine zweite Führung für einen zweiten Leiter zum Anschluss an den Anschlussbereich (10) des zweiten Anschlusselements (8) gebildet ist.

3. Baugruppe (1) nach Anspruch 2, wobei die erste Führung benachbart zu dem Anschlussbereich (9) des ersten Anschlusselements (7) gebildet ist und / oder wobei die zweite Führung benachbart zu dem Anschlussbereich (10) des zweiten Anschlusselements (8) gebildet ist.

4. Baugruppe (1) nach einem der vorangehenden Ansprüche, wobei das erste Anschlusselement (7) und das zweite Anschlusselement (8) zueinander versetzt angeordnet sind.

5. Baugruppe (1) nach einem der vorangehenden Ansprüche weiter eine Antenne (26) aufweisend, wobei die Antenne (26) in einem Zwischenbereich, der zwischen dem ersten Anschlusselement (7) und dem zweiten Anschlusselement (8) gebildet ist, einen U-förmigen Verlauf (27) hat.

6. Baugruppe (1) nach einem der vorangehenden Ansprüche ferner eine weitere Aufnahmeeinrichtung (13) für einen weiteren Stecker aufweisend, wobei in einem vorderen Bereich der weiteren Aufnahmeeinrichtung ein drittes Anschlusselement angeordnet ist, und wobei in einem hinteren Bereich der weiteren Aufnahmeeinrichtung ein viertes Anschlusselement angeordnet ist.

7. Elektrodenanschlussvorrichtung für ein Implantat mit einer Baugruppe (1) nach einem der vorangehenden Ansprüche.

8. Implantat mit einer Elektrodenanschlussvorrichtung nach Anspruch 7.

9. Verfahren zum Bilden einer Elektrodenanschlussvorrichtung eines Implantats, mit folgenden Schritten:
- Bereitstellen einer Baugruppe (1) gemäß Anspruch 1,
- Anordnen und Befestigen eines Federelements (20) in der Aufnahmeeinrichtung (2) der Baugruppe (1),
- Verschließen von Öffnungen (2, 12b) der Aufnahmeeinrichtung mit Vergusshilfsmitteln (21, 22, 23),
- Befestigen eines ersten Leiters an dem ersten Anschlusselement (7) der Baugruppe (1),
- Befestigen eines zweiten Leiters an dem zweiten Anschlusselement (8) der Baugruppe (1),
- Anordnen der Baugruppe (1) auf einem Gehäuse (29) des Implantats,
- Verbinden des ersten Leiters mit einer Durchführung (30), die an dem Gehäuse (29) gebildet ist,
- Verbinden des zweiten Leiters mit der Durchführung (30),
- Anordnen der Baugruppe (1) mit dem Gehäuse (29) in einer Gießform,
- Füllen der Gießform mit einem Kunstharz (31),
- nach Aushärten des Kunstharzes (31), Entfernen der Vergusshilfsmittel (21, 22, 23).

10. Verfahren nach Anspruch 9, die weiteren Schritte umfassend:
- Anordnen und Befestigen einer Antenne (26) an der Baugruppe (1),
- Verbinden der Antenne (26) mit der Durchführung (30),
wobei die weiteren Schritte ausgeführt werden, bevor die Baugruppe (1) auf dem Gehäuse (29) angeordnet wird.

## Claims

1. An assembly (1) for an electrode connection device of an implant, comprising:
- a continuous receiving unit (2) for a plug;
- a first connecting element (7), which is arranged in a front region of the receiving unit (2), wherein the first connecting element (7) includes at least two flat lateral surfaces; and
- a second connecting element (8), which is arranged in a rear region of the receiving unit (2), wherein the second connecting element (8) includes at least two flat lateral surfaces;
wherein
the assembly (1) is at surrounded by a plastic material (11) at least in sections, and a connecting region (9) of the first connecting element (7) is free of plastic material (11) and a connecting region (10) of the second connecting element (8) is free of plastic material (11), and
a positioning unit (18b) is formed at a rear end of the receiving unit.

2. The assembly (1) according to claim 1, wherein a first guide for a first conductor for connection to the connecting region (9) of the first connecting element (7) is formed in the plastic material (11) and/or wherein a second guide for a second conductor for connection to the connecting region (10) of the second connecting element (8) is formed in the plastic material (11).

3. The assembly (1) according to claim 2, wherein the first guide is formed so as to adjoin the connecting region (9) of the first connecting element (7) and/or wherein the second guide is formed so as to adjoin the connecting region (10) of the second connecting element (8).

4. The assembly (1) according to any one of the preceding claims, wherein the first connecting element (7) and the second connecting element (8) are arranged offset from one another.

5. The assembly (1) according to any one of the preceding claims, further comprising an antenna (26), the antenna (26) having a U-shaped progression (27) in an intermediate region, which is formed between the first connecting element (7) and the second connecting element (8).

6. The assembly (1) according to any one of the preceding claims, further comprising a further receiving unit (13) for a further plug, a third connecting element being arranged in a front region of the further receiving unit, and a fourth connecting element being arranged in a rear region of the further receiving unit.

7. An electrode connection device for an implant, comprising an assembly (1) according to any one of the preceding claims.

8. An implant comprising an electrode connection device according to claim 7.

9. A method for forming an electrode connection device of an implant, comprising the following steps:
- providing an assembly (1) according to claim 1;
- arranging and attaching a spring element (20) in the receiving unit (2) of the assembly (1);
- sealing openings (2, 12b) of the receiving unit using potting aids (21, 22, 23);
- attaching a first conductor to the first connecting element (7) of the assembly (1);
- attaching a second conductor to the second connecting element (8) of the assembly (1);
- arranging the assembly (1) on a housing (29) of the implant;
- connecting the first conductor to a feedthrough (30) which is formed on the housing (29);
- connecting the second conductor to the feedthrough (30);
- arranging the assembly (1), together with the housing (29), in a casting mold;
- filling the casting mold with a synthetic resin (31); and
- after the synthetic resin (31) has cured, removing the potting aids (21, 22, 23).

10. The method according to claim 9, comprising the further steps:
- arranging and attaching an antenna (26) to the assembly (1); and
- connecting the antenna (26) to the feedthrough (30),
wherein the further steps are carried out before the assembly (1) is arranged on the housing (29).

## Revendications

1. Ensemble (1) pour un dispositif de connexion d'électrodes d'un implant, comprenant :
- une unité de réception continue (2) pour une prise ;
- un premier élément de connexion (7), qui est agencé dans une région avant de l'unité de réception (2), dans lequel le premier élément de connexion (7) inclut au moins deux surfaces latérales plates ; et
- un deuxième élément de connexion (8), qui est agencé dans une région arrière de l'unité de réception (2), dans lequel le deuxième élément de connexion (8) inclut au moins deux surfaces latérales plates ;
dans lequel
l'ensemble (1) est entouré d'une matière plastique (11) au moins par sections, et une région de connexion (9) du premier élément de connexion (7) est exempte de matière plastique (11) et une région de connexion (10) du deuxième élément de connexion (8) est exempte de matière plastique (11), et
une unité de positionnement (18b) est formée au niveau d'une extrémité arrière de l'unité de réception.

2. Ensemble (1) selon la revendication 1, dans lequel un premier guide pour un premier conducteur pour connexion à la région de connexion (9) du premier élément de connexion (7) est formé dans la matière plastique (11) et/ou dans lequel un second guide pour un second conducteur pour connexion à la région de connexion (10) du deuxième élément de connexion (8) est formé dans la matière plastique (11).

3. Ensemble (1) selon la revendication 2, dans lequel le premier guide est formé de sorte à s'accoupler à la région de connexion (9) du premier élément de connexion (7) et/ou dans lequel le second guide est formé de manière à s'accoupler à la région de connexion (10) du deuxième élément de connexion (8).

4. Ensemble (1) selon l'une quelconque des revendications précédentes, dans lequel le premier élément de connexion (7) et le deuxième élément de connexion (8) sont agencés de manière décalée l'un par rapport à l'autre.

5. Ensemble (1) selon l'une quelconque des revendications précédentes, comprenant en outre une antenne (26), l'antenne (26) ayant une progression en U (27) dans une région intermédiaire, qui est formée entre le premier élément de connexion (7) et le deuxième élément de connexion (8).

6. Ensemble (1) selon l'une quelconque des revendications précédentes, comprenant en outre une unité de réception supplémentaire (13) pour une prise supplémentaire, un troisième élément de connexion agencé dans une région avant de l'unité de réception supplémentaire, et un quatrième élément de connexion agencé dans une région arrière de l'unité de réception supplémentaire.

7. Dispositif de connexion d'électrodes pour un implant, comprenant un ensemble (1) selon l'une quelconque des revendications précédentes.

8. Implant comprenant un dispositif de connexion d'électrodes selon la revendication 7.

9. Procédé de formation d'un dispositif de connexion d'électrodes d'un implant, comprenant les étapes suivantes consistant à :
- fournir un ensemble (1) selon la revendication 1 ;
- agencer et attacher un élément de ressort (20) dans l'unité de réception (2) de l'ensemble (1) ;
- sceller les ouvertures (2, 12b) de l'unité de réception en utilisant des aides d'enrobage (21, 22, 23) ;
- attacher un premier conducteur au premier élément de connexion (7) de l'ensemble (1) ;
- attacher un second conducteur au deuxième élément de connexion (8) de l'ensemble (1) ;
- agencer l'ensemble (1) sur un boîtier (29) de l'implant ;
- connecter le premier conducteur à une connexion d'interface (30) qui est formée sur le boîtier (29) ;
- connecter le second conducteur à la connexion d'interface (30) ;
- agencer l'ensemble (1), conjointement au boîtier (29), dans un moule de coulée ;
- remplir le moule de coulée d'une résine synthétique (31) ; et
- après que la résine synthétique (31) a durci, retirer les aides d'enrobage (21, 22, 23).

10. Procédé selon la revendication 9, comprenant les étapes supplémentaires consistant à :
- agencer et attacher une antenne (26) à l'ensemble (1) ; et
- connecter l'antenne (26) à la connexion d'interface (30),
dans lequel les étapes supplémentaires sont mises en œuvre avant que l'ensemble (1) ne soit agencé sur le boîtier (29).
